(19) Europäisches Patentamt
European Patent Office
Office européen des brevets

(11) EP 4 755 947 A1

(12) # EUROPEAN PATENT APPLICATION
published in accordance with Art. 153(4) EPC

(43) Date of publication:
**10.06.2026 Bulletin 2026/24**

(21) Application number: **24848370.3**

(22) Date of filing: **02.08.2024**

(51) International Patent Classification (IPC):
***C08G 73/18*** *(2006.01)* ***G01N 21/64*** *(2006.01)*
***C09K 9/02*** *(2006.01)*

(52) Cooperative Patent Classification (CPC):
**B01D 53/02; B01J 20/26; B01J 20/30; C07D 235/04; C08G 73/18; C09K 9/02; G01N 21/64;** Y02E 60/50

(86) International application number:
**PCT/CN2024/109374**

(87) International publication number:
**WO 2025/026416 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.08.2023 CN 202310967288**

(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **LI, Jing**
  **Beijing 100013 (CN)**
- **WANG, Ailian**
  **Beijing 100013 (CN)**
- **ZHANG, Taoyi**
  **Beijing 100013 (CN)**
- **JI, Wenxi**
  **Beijing 100013 (CN)**
- **WU, Jianing**
  **Beijing 100013 (CN)**
- **ZHANG, Longgui**
  **Beijing 100013 (CN)**
- **NIU, Xinmiao**
  **Beijing 100013 (CN)**
- **SONG, Jianhui**
  **Beijing 100013 (CN)**
- **SU, Cui**
  **Beijing 100013 (CN)**
- **DU, Wenjie**
  **Beijing 100013 (CN)**

(74) Representative: **Hoffmann Eitle**
**Patent- und Rechtsanwälte PartmbB**
**Arabellastraße 30**
**81925 München (DE)**

(54) **USE OF POLYBENZIMIDAZOLE AS MECHANOCHROMIC MATERIAL**

(57) The invention relates to the technical field of mechanochromic materials, and discloses use of polybenzimidazole as a mechanochromic material.

EP 4 755 947 A1

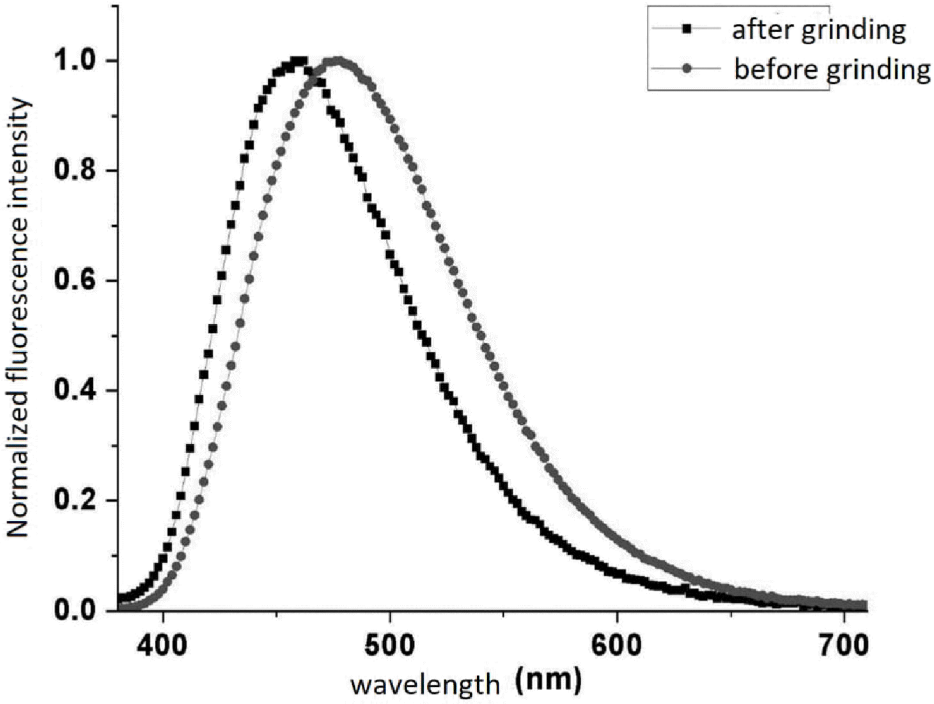
after grinding
before grinding
Normalized fluorescence intensity
0.0
0.2
0.4
0.6
0.8
1.0
400
500
600
700
wavelength (nm)

FIG.3

## Description

### Technical Field

The invention relates to the field of mechanochromic materials, in particular to use of polybenzimidazole as mechanochromic material.

### Backgrounds

A mechanochromic material is a class of materials which undergoes change in color property, luminescence property or the like of the material when subjected to an external force stimulus, such as a stimulus of grinding, stretching, compressing or other stimuli. The mechanochromic material exhibits wide application prospects in various fields such as mechanical sensing, secure communication, nondestructive inspection of material, human motion monitoring and the like.

The existing mechanochromic materials include a large proportion of small molecule-type mechanochromic materials and a small proportion of polymer-type mechanochromic materials. The processability, mechanical strength and other characteristics of polymers enable a much wider application range of polymers. Mechanochromic polymer materials typically incorporate color-changing molecules into polymers by way of blending or chemical bond linking. The reported polymer materials with incorporation of color-changing groups by chemical bonds include polymers containing spiropyran, rhodamine, or diarylbenzofuranone structures, which generally have low melting points or glass transition temperatures, have poor heat resistance, and cannot meet the requirements for use under high-temperature conditions.

### Summary of Invention

The invention aims to provide a polymer material with mechanochromic property, which has high thermal decomposition temperature/thermal stability and high mechanical property so as to meet the requirement of using under high temperature and/or high mechanical property conditions.

The inventors of the present invention have unexpectedly found that polybenzimidazole polymers per se have mechanochromic property, without the need to dope mechanochromic small molecules into polymer or to attach mechanochromic groups into or onto polymer molecular chains through chemical bonds or non-chemical bonds. More specifically, the inventors of the present invention have unexpectedly found that the fluorescence emission peak of a polybenzimidazole polymer is significantly shifted under an external force stimulus.

Accordingly, the present invention provides the use of polybenzimidazole polymer as mechanochromic material.

The polybenzimidazole polymer can be used as a mechanochromic material in the applications of mechanical sensing, communication, anti-counterfeiting, nondestructive inspection of material, motion detection, micro-flow monitoring and the like.

The invention provides the following advantageous effects:

(1) the invention finds that polybenzimidazole has the mechanochromic property, which widens the application fields of polybenzimidazole;
(2) the mechanochromic materials in prior art mostly produce a red shift in fluorescence emission peak after being stimulated by an external force, while some embodiments of the present invention provide polybenzimidazoles which produce a blue shift in fluorescence emission peak after being stimulated by an external force;
(3) polybenzimidazole materials have excellent thermal stability and high mechanical strength, which widens the application scenarios of mechanochromic materials.

### Description of Drawings

FIG. 1 is hydrogen nuclear magnetic resonance spectra of the polymer of Example 1 (PBI-1) and the polymer of Example 7 (PBI-7);
FIG. 2 is a thermogravimetric analysis diagram of the polymer of Example 1 (PBI-1);
FIG. 3 is fluorescence spectra of the polymer of Example 2 (PBI-2) before and after grinding; and
FIG. 4 is fluorescence spectra of the polymer film product of Example 9 (PBI-9) before and after stretching.

### Detailed Description

The endpoints of the ranges and any values disclosed herein are not limited to the precise ranges or values, and

these ranges or values should be understood to encompass values close to these ranges or values. For numerical ranges, the endpoint values of the various ranges, the endpoint values of the various ranges and individual point values, and individual point values can be combined with each other to give one or more new numerical ranges, and such numerical ranges should be construed as concretely disclosed herein.

The first aspect of the present invention provides the use of polybenzimidazole as a mechanochromic material. The present application finds that polybenzimidazole has mechanochromic property. In the present invention, the polybenzimidazole can be any polybenzimidazole polymer known in the art.

According to the present invention, in some embodiments, the polybenzimidazole comprises structural unit A, the structural unit A having a structure represented by formula (1):

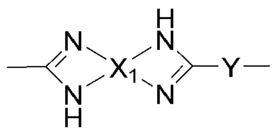
formula (1)

wherein, the $X_1$ group is a linking group provided by an aromatic hydrocarbon compound containing 1-10 benzene rings; and the Y group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$ and Y groups are each independently not substituted or substituted by at least one substituent selected from the group consisting of hydroxyl, amino, cyano, halogen, $C_1$-$C_4$ alkyl, and $C_1$-$C_4$ haloalkyl. In some embodiments, the polybenzimidazole consists of structural unit A.

According to the present invention, in some embodiments, the polybenzimidazole comprises structural unit A and structural unit C, comprises structural unit A and structural unit D, or comprises structural unit A, structural unit B, structural unit C and structural unit D; wherein the structural unit A has a structure represented by formula (1), the structural unit B has a structure represented by formula (2), the structural unit C has a structure represented by formula (1-1), and the structural unit D has a structure represented by formula (2-2):

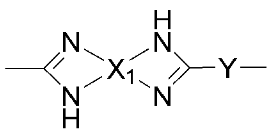
formula (1)
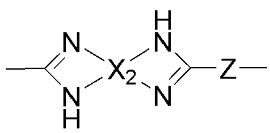
formula (2)

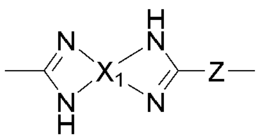
formula (1-1)
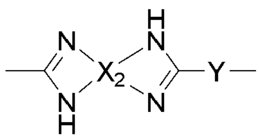
formula (2-2)

wherein, the $X_1$ and $X_2$ groups are each independently a linking group provided by an aromatic hydrocarbon compound containing 1-10 benzene rings; the Y and Z groups are each independently a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl. In some embodiments, the polybenzimidazole consists of structural unit A and structural unit C. In some embodiments, the polybenzimidazole consists of structural unit A and structural unit D. In some embodiments, the polybenzimidazole consists of structural unit A, structural unit B, structural unit C, and structural unit D.

According to the present invention, in some embodiments, the polybenzimidazole comprises or consists of structural unit A and optionally structural unit, the structural unit A having a structure represented by formula (1) and the structural unit B having a structure represented by formula (2);

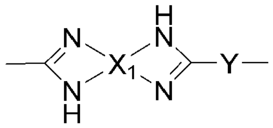 formula (1) 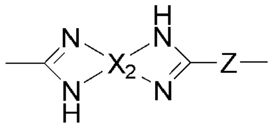 formula (2)

wherein, the $X_1$ and $X_2$ groups are each independently a linking group provided by an aromatic hydrocarbon compound containing 1-10 benzene rings; the Y and Z groups are each independently a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin, and Y is different from Z when the structural unit B exists; wherein, the $X_1$, $X_2$, Y and/or Z groups are each independently not substituted or substituted by at least one substituent selected from the group consisting of hydroxyl, amino, cyano, halogen, $C_1$-$C_4$ alkyl, and $C_1$-$C_4$ haloalkyl.

In the present invention, the linking ways of the $X_1$ and $X_2$ groups in formula (1) and formula (2) are known to those skilled in the art.

For example, for formula (1) and formula (2), when the $X_1$ and $X_2$ groups are each independently a linking group provided by benzene, then the structural formulas of formula (1) and formula (2) can be respectively

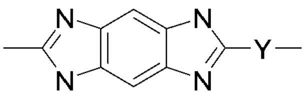 and 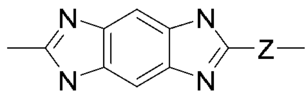 ;

that is, the benzene ring and imidazole rings share two pairs of carbon atoms. Any two pairs of ortho-position carbon atoms on benzene rings in the $X_1$ and $X_2$ groups participate in the formation of imidazole rings, and the two pairs of ortho-position carbon atoms can be from the same benzene ring or can be from different benzene rings in a plurality of benzene rings. For example, when the $X_1$ group is a linking group provided by biphenyl, each benzene ring of biphenyl can respectively provide one pair of carbon atoms to form imidazole ring, or alternatively one benzene ring can provide two pairs of ortho-position carbon atoms to form imidazole rings.

In the present invention, $C_4$-$C_{10}$ cycloalkane can be, for example, cyclobutane, cyclopentane, cyclohexane, cycloheptane, cyclooctane, cyclononane or cyclodecane.

In the present invention, $C_1$-$C_8$ paraffinic hydrocarbon can be, for example, methane, ethane, n-propane, iso-propane, butane, pentane, hexane, heptane, or octane. In some embodiments, $C_1$-$C_8$ paraffinic hydrocarbon can also be a $C_1$-$C_8$ paraffinic hydrocarbon substituted by one or more halogen atoms, wherein the halogen atom can be selected from the group consisting of fluorine atom, chlorine atom and bromine atom.

In the present invention, $C_2$-$C_8$ olefin can be, for example, ethylene, propylene, butene, pentene, hexene, heptene, or octene.

In the present invention, halogen can be selected from, for example, fluorine, chlorine and bromine.

In the present invention, $C_1$-$C_4$ alkyl can be, for example, methyl, ethyl, n-propyl, isopropyl, n-butyl, sec-butyl, isobutyl, or tert-butyl.

In the present invention, $C_1$-$C_4$ haloalkyl can be, for example, halomethyl, haloethyl, halo-n-propyl, halo-isopropyl, halo-n-butyl, halo-sec-butyl, halo-isobutyl, or halo-tert-butyl; the halogen atom(s) in the $C_1$-$C_4$ haloalkyl can be, for example, selected from fluorine atom, chlorine atom and bromine atom.

According to the present invention, preferably, in some embodiments, the $X_1$ and $X_2$ groups are each independently a linking group provided by at least one compound selected from the group consisting of benzene, biphenyl, terphenyl, a bridged-benzenes, an iptycene, and a fused ring aromatic hydrocarbon containing 1-10 benzene rings; more preferably, the $X_1$ and $X_2$ groups are each independently a linking group provided by at least one compound selected from the group consisting of benzene, biphenyl, a bridged-benzenes, an iptycene, and naphthalene; further preferably, the bridged-benzenes has a structure represented by formula (3), formula (4) or formula (5), wherein $R_1$, $R_3$, $R_4$ are each independently selected from the group consisting of oxygen atom, sulfur atom, carbonyl, sulfonyl, methylene, and halogen-substituted methylene; and $R_2$ is selected from the group consisting of heteroatom-containing aromatic rings;

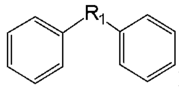 formula (3) 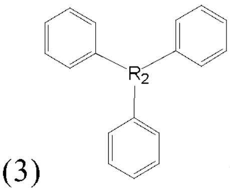 formula (4) 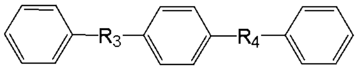 formula (5).

In the present invention, the heteroatom-containing aromatic ring can be, for example, pyridine or pyrimidine.

In the present invention, the linking positions (indicated by "dotted line") of the structures represented by formulas (3), (4) and (5) can be as follows:

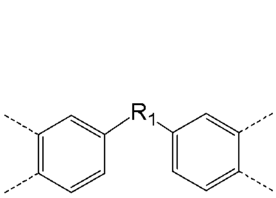, 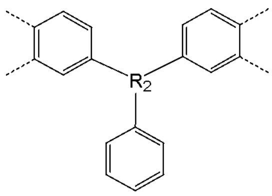, 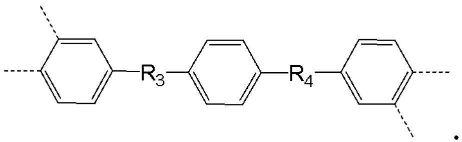.

In the present invention, the linking positions include, but not limited to, the above linking positions; for example, any two adjacent linking positions on the same benzene ring are possible.

According to the present invention, preferably, in some embodiments, the $X_1$ and $X_2$ groups are each independently selected from the following linking groups:

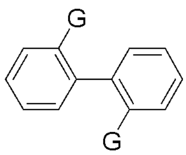 、 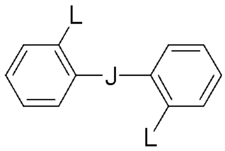 、 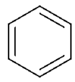 、 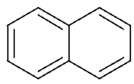 、

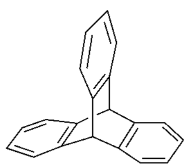 、 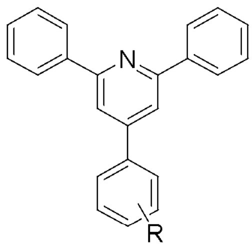 、 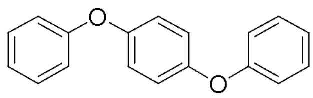 ;

wherein G is selected from the group consisting of hydrogen atom, methyl and trifluoromethoxy; J is selected from the group consisting of oxygen atom, sulfur atom, carbonyl, sulfonyl, methylene and methyl- or trifluoromethyl-substituted methylene; L is selected from the group consisting of bromine atom, phenyl and trifluoromethyl-substituted phenyl; and R is selected from the group consisting of hydrogen atom, carboxyl, hydroxyl and trifluoromethyl.

In the present invention, in some embodiments, the linking positions in the $X_1$ and $X_2$ groups can include, but not limited to, the linking positions shown below:

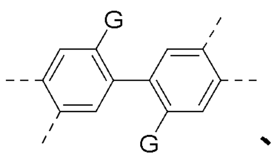 、 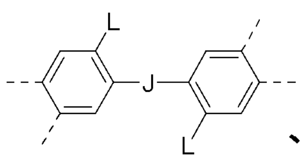 、 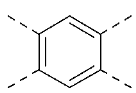 、 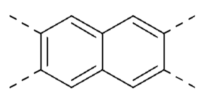 、

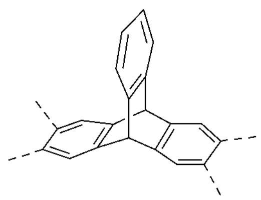, 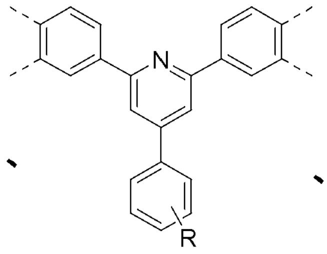, 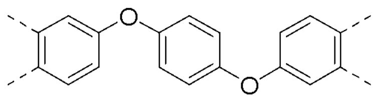.

According to present invention, in some embodiments, the Y and Z groups are each independently a linking group provided by at least one compound selected from the group consisting of benzene, a 5-6 membered nitrogen-containing heterocyclic compound, an aromatic hydrocarbon compound containing 2-7 benzene rings, an aromatic hydrocarbon compound containing 1-2 nitrogen-containing heterocyclic rings and 1-5 benzene rings, a $C_4$-$C_{10}$ cycloalkane, a $C_1$-$C_8$ paraffinic hydrocarbon, and a $C_2$-$C_8$ olefin.

According to the present invention, in some embodiments, the molar content ratio of the $X_2$ group to $X_1$ group can be 0-9999, preferably 0-99; for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, or a range formed by any two of above values. In the present invention, in the absence of specific description, the content ratio of the $X_2$ structural unit or group to $X_1$ structural unit or group in polybenzimidazole is calculated from the feeding amounts.

According to the present invention, preferably, in some embodiments, the Y and Z groups can each independently be a linking group selected from:

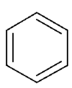, 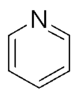, 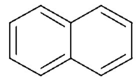, 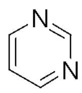, 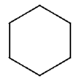, 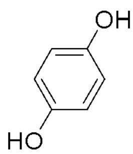, 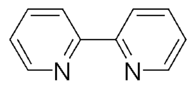, 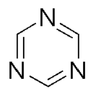,

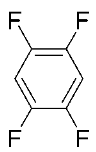, 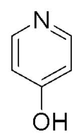, 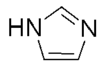, 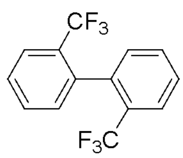, 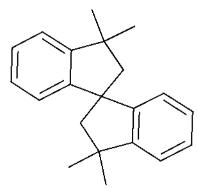, 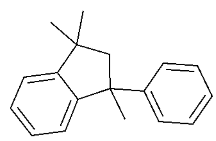,

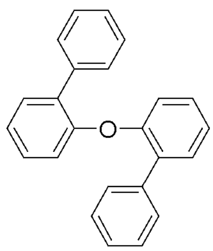, 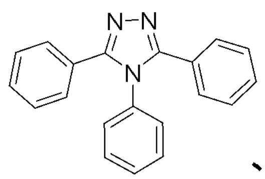, 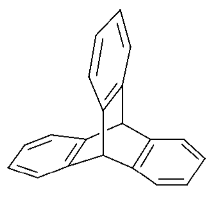, 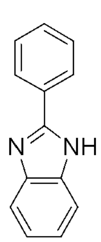, 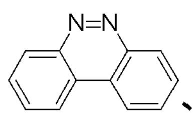,

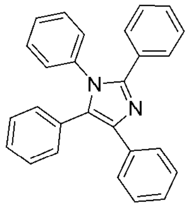, 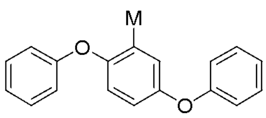, 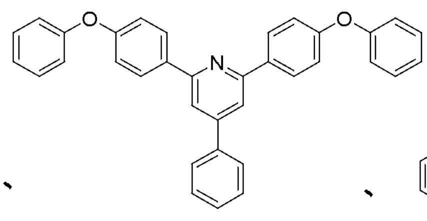, 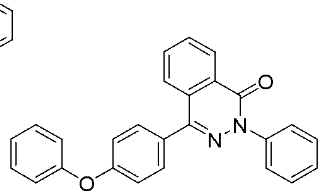,

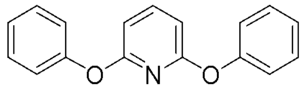 、 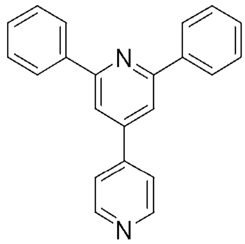 、 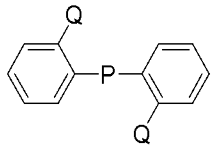 、 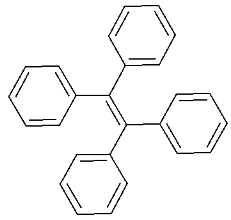 、

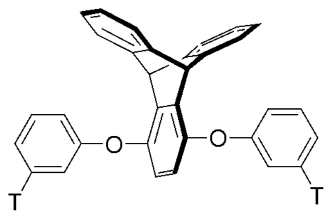 、 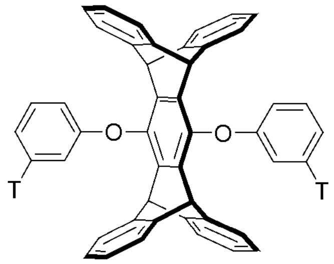 、 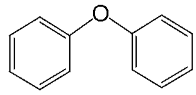 、

$C_1$-$C_8$ paraffinic hydrocarbon group;

wherein M is selected from the group consisting of hydrogen atom, cyano, methyl, phenyl and methyl- or trifluoromethyl-substituted phenyl; P is selected from the group consisting of oxygen atom, sulfur atom, carbonyl, sulfonyl, cyclobutyl, halogen-substituted cyclobutyl, methylene and halogen-substituted methylene; Q is selected from the group consisting of bromine atom, phenyl and trifluoromethyl-substituted phenyl; and T is selected from the group consisting of hydrogen atom and methyl.

In the present invention, there are no particular limits to the linking positions of the Y and Z groups, and the linking positions can be any position on the linking group. For example, when the Y and Z groups contain a cyclic structure, the linking position can be a carbon atom on the cyclic structure; when the Y and Z groups are $C_1$-$C_8$ paraffinic hydrocarbon groups, the linking position can be any carbon atom in the paraffinic hydrocarbon group. In the present invention, the linking positions of the Y and Z groups can include, but not limited to, the linking positions shown below:

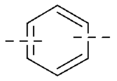 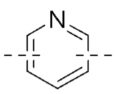 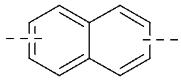 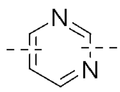 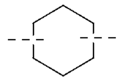 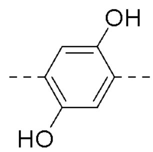 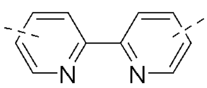 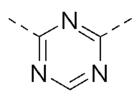

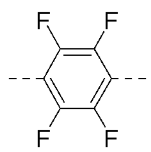 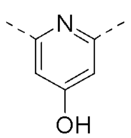 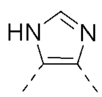 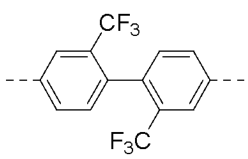 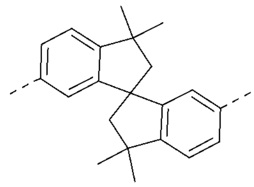 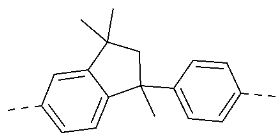

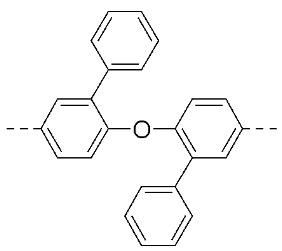 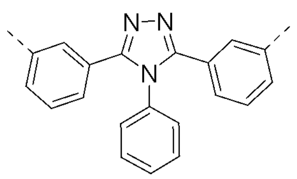 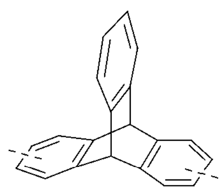 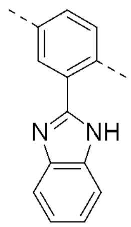 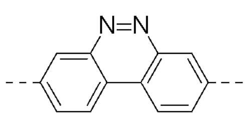

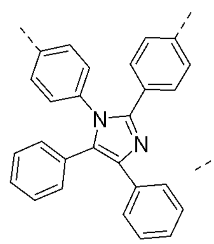
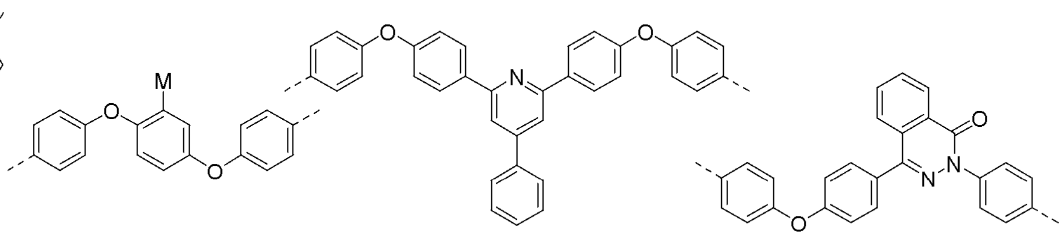
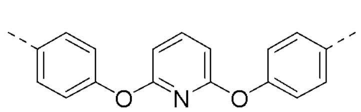
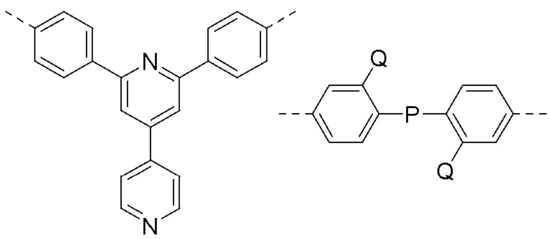
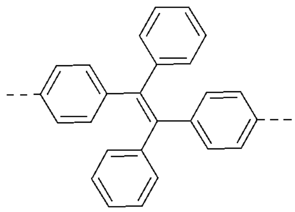
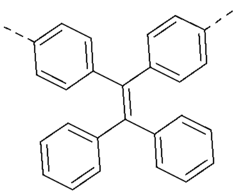
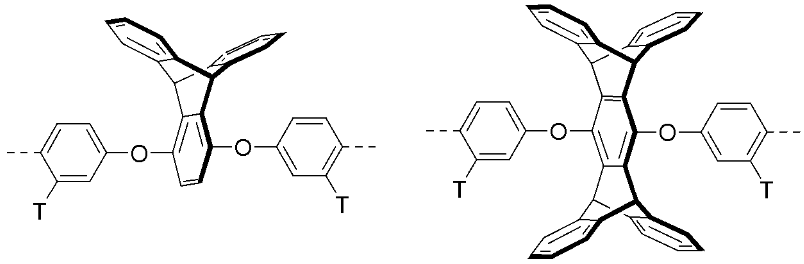.

In some embodiments, the polybenzimidazole polymer of the present invention does not contain mechanochromic groups generally known in the art, wherein the mechanochromic groups including but not limited to mechanochromic groups derived from tetraphenylethylene, triarylamine, spiropyran, rhodamine, coumarin, and the like.

In the present invention, there are no particularly limits to the molecular weight of the polybenzimidazole. The molecular weight of the polybenzimidazole can be selected, for example, according to the requirements of a specific application.

According to the present invention, in some embodiments, the polybenzimidazole can have an intrinsic viscosity of 0.15 to 3.0dL $g^{-1}$; for example, the intrinsic viscosity can be 0.15 dL $g^{-1}$, 0.2 dL $g^{-1}$, 0.3dL $g^{-1}$, 0.4dL $g^{-1}$, 0.8dL $g^{-1}$, 1.2dL $g^{-1}$, 1.6dL $g^{-1}$, 1.7dL $g^{-1}$, 1.8dL $g^{-1}$, 1.9dL $g^{-1}$, 2dL $g^{-1}$, 2.1dL $g^{-1}$, 2.2dL $g^{-1}$, 2.3dL $g^{-1}$, 2.4dL $g^{-1}$, 2.5dL $g^{-1}$, 2.6dL $g^{-1}$, 2.7dL $g^{-1}$, 2.8dL $g^{-1}$, 2.9 dL $g^{-1}$, 3.0 dL $g^{-1}$, and a range formed by any two of above values.

According to the present invention, in some embodiments, the molar content ratio of the Z group to Y group can be 0-9999, preferably 0-99; for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, or a range formed by any two of above values. In the present invention, in the absence of specific description, the content ratio of Y structural unit or group to Z structural unit or group in polybenzimidazole is calculated from the feeding amounts.

In some embodiments of the invention, a polybenzimidazole is provided which exhibits a blue shift in fluorescence emission peak after stimulated with an external force.

In some embodiments of the invention, the polybenzimidazole is poly(2,5-benzimidazole).

Polybenzimidazoles are polymers known in the art. The present invention can use polybenzimidazole polymers of various structures known in the art.

The preparation of polybenzimidazoles is known in the art. Polybenzimidazoles can be prepared using various preparation methods known in the art.

In some embodiments, the present invention also provides a method for preparing the polybenzimidazole of the first aspect, the method comprising reacting at least one compound having a structure represented by formula (6) with HOOC-Y-COOH and optionally HOOC-Z-COOH;

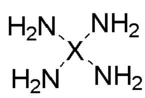 formula (6).

The X group in formula (6) is the same as the $X_1$ and/or $X_2$ group in formula (1) and formula (2), and thus is not described here again.

According to the preparing method of the present invention, preferably, in some embodiments, the compound having the structure represented by formula (6) is at least one selected from the following compounds:

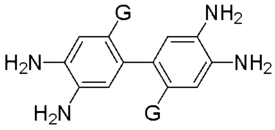 、 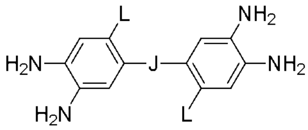 、 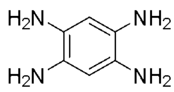 、 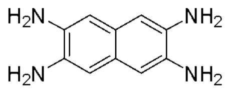 、

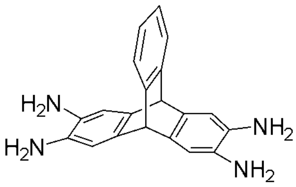 、 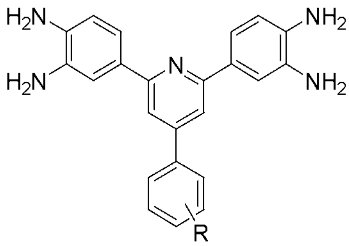 、 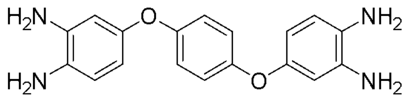 ;

wherein, G is selected from the group consisting of hydrogen atom, methyl and trifluoromethoxy, i.e. -H , $-CH_3$, $-OCF_3$ ; J is selected from the group consisting of oxygen atom, sulfur atom, carbonyl, sulfonyl, methylene, and methyl- or trifluoromethyl-substituted methylene, such as

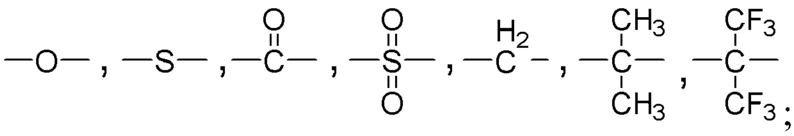

L is selected from the group consisting of bromine atom, phenyl and trifluoromethyl-substituted phenyl, such as

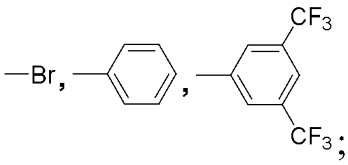

and

R is selected from the group consisting of hydrogen atom, carboxyl, hydroxyl and trifluoromethyl, i.e., -H , -COOH , -OH , $-CF_3$.

According to the preparing method of the present invention, preferably, in some embodiments, HOOC-Y-COOH and optionally HOOC-Z-COOH are each independently selected from at least one of the following compounds:

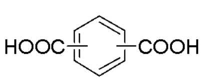 、 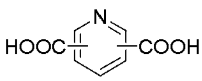 、 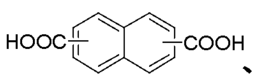 、 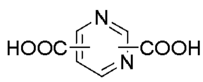 、 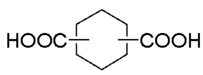 、

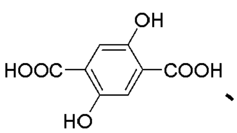 、 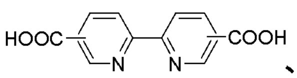 、 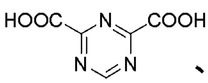 、 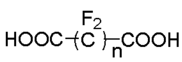 、 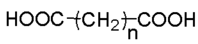 、

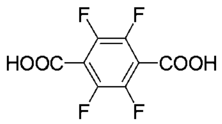 、 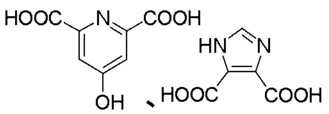 、 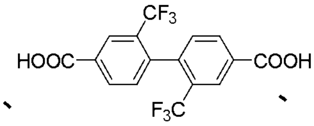 、 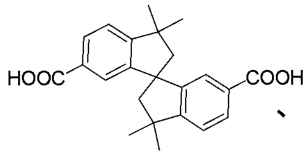 、

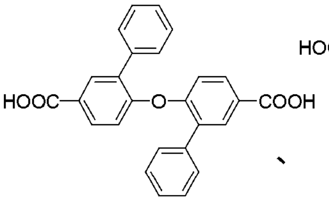 、 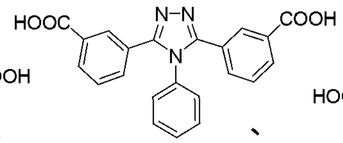 、 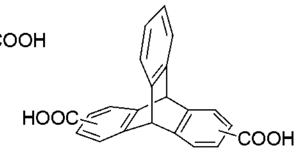 、 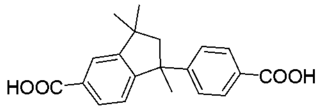 、

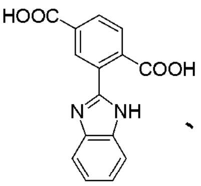 、 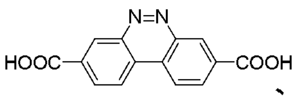 、 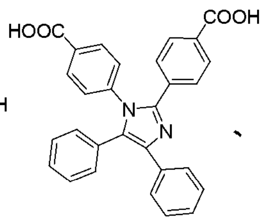 、 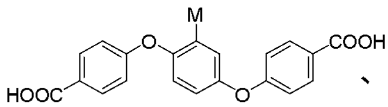 、

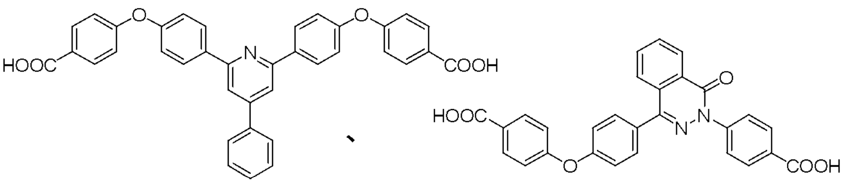 、 、 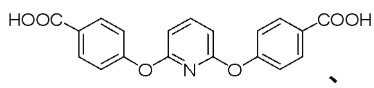 、

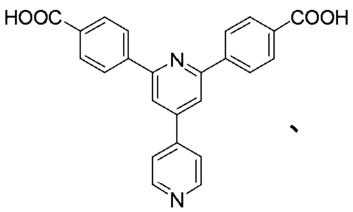 、 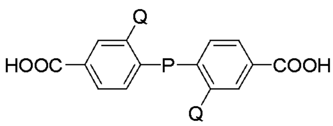 、 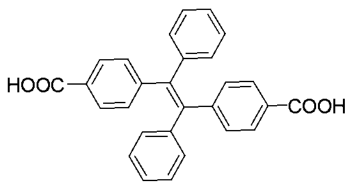 、

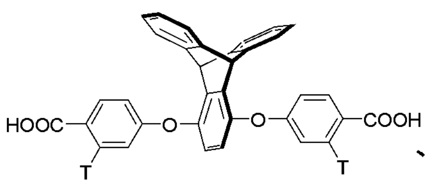 、 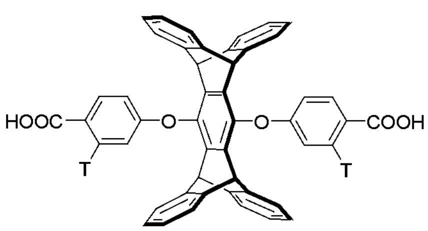 、 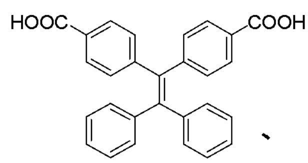 、

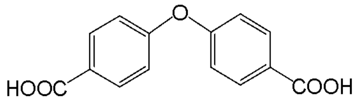 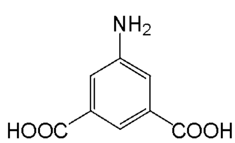 ;

wherein, n = 1-8;

M is selected from the group consisting of hydrogen atom, cyano, methyl, phenyl and methyl- or trifluoromethyl-substituted phenyl, for example

—H, —CN, 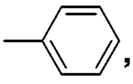, 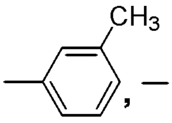, —$CH_3$, 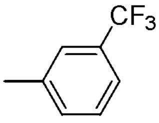;

P is selected from the group consisting of oxygen atom, sulfur atom, carbonyl, sulfonyl, cyclobutyl, halogen-substituted cyclobutyl, methylene and methyl- or trifluoromethyl-substituted methylene, such as

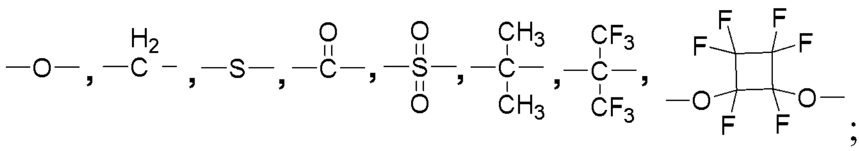

Q is selected from the group consisting of bromine atom, phenyl and trifluoromethyl-substituted phenyl, such as

—Br, 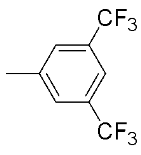;

and
T is selected from the group consisting of hydrogen atom and methyl, i.e. -H, -$CH_3$.

According to the preparing method of the present invention, preferably, in some embodiments, the reaction is performed in a solvent, more preferably, the solvent is polyphosphoric acid or a mixed solution of methanesulfonic acid/phosphorus pentoxide, further preferably a mixed solution of methanesulfonic acid/phosphorus pentoxide and the mass of phosphorus pentoxide is 2-10% of the mass of methanesulfonic acid. The concentration of the mixed monomers in the reaction system can be 3 wt% to 20 wt%.
According to the preparing method of the present invention, preferably, in some embodiments, the ratio of the molar amount of the compound having the structure represented by formula (6) to the total molar amount of HOOC-Y-COOH and optionally HOOC-Z-COOH is 1:0.6-2, preferably 1:0.8-1.2.
In some embodiments, 3,4-diaminobenzoic acid is used as the monomer to prepare poly(2,5-benzimidazole).
According to the preparing method of the present invention, preferably, in some embodiments, the reaction time can be 1 to 8 hours, preferably 3 to 5 hours. The reaction temperature can be 60 to 230 °C, preferably 60 to 170 °C, more preferably 100 °C to 150 °C. The reaction pressure can be atmospheric pressure.
According to the preparing method of the present invention, preferably, in some embodiments, the ratio of the molar amount of the compound having the structure represented by formula (6) to the total molar amount of HOOC-Y-COOH and optionally HOOC-Z-COOH is 1:0.6-2; for example, 1:0.6, 1:0.7, 1:0.8, 1:0.9, 1:1, 1:1.1, 1:1.2, 1:1.3, 1:1.4, 1:1.5, 1: 1.6, and a range formed by any two of above values; preferably 1:0.8-1.2; wherein the molar content ratio of the $X_2$ group to the $X_1$ group can be 0-9999, preferably 0-99, and for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, and a range formed by any two of above values; the molar content ratio of Z group to Y group can be 0-9999, preferably 0-99, and for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, and a range formed by any two of above values.
In some embodiments, the polybenzimidazole of the present invention can have one of the structures shown below:

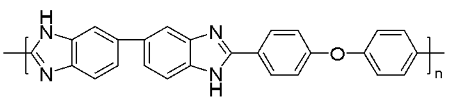、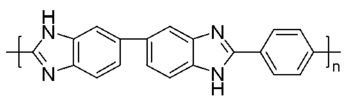、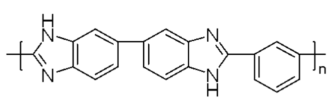

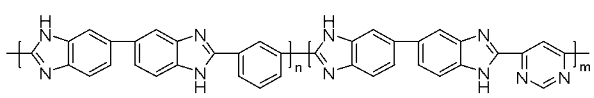、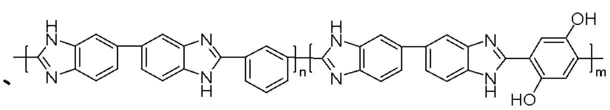

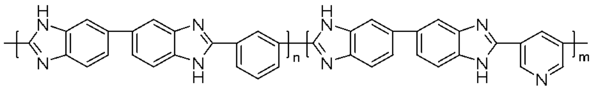
、
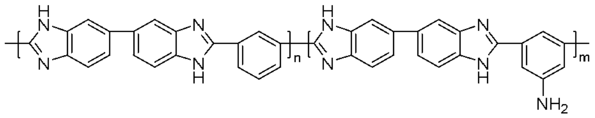

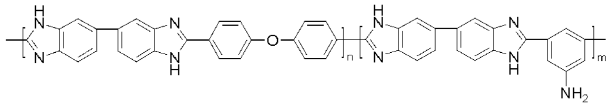
.

In the structures shown above, with respect to copolymers, the structures merely illustrate that the polymer chains comprise the shown repeating structural units or that the polymer chains consist of the shown repeating structural units, and do not illustrate the distributions of the shown repeating structural units in the polymer chains.

It is found in present invention that polybenzimidazole has the mechanochromic property, that is, the fluorescence emission peak of polybenzimidazole is significantly shifted (red shift or blue shift) under the stimulus of an external force. The stimulus of external force includes, but not limited to, mechanical actions such as grinding, stretching, compressing, impacting and the like. This finding is unexpected because polymers having mechanochromic property in the prior art either require doping of mechanochromic small molecules or require attaching mechanochromic groups into or onto polymer molecular chains by chemical or non-chemical bonds.

In some embodiments, the present application provides the use of polybenzimidazole as mechanochromic material, which can include the step of measuring the fluorescence emission spectrum of polybenzimidazole after the polybenzimidazole has been subjected to an action of external force. Further, the use can further include a step of comparing the position of the fluorescence emission peak of the fluorescence emission spectrum of polybenzimidazole measured after being subjected to the action of external force with the position of the fluorescence emission peak of the fluorescence emission spectrum of polybenzimidazole before being subjected to the action of external force. The use can also issue an alert signal according to the result of comparison. For example, if the shift of the fluorescence emission peak of the fluorescence emission spectra before and after the action of external force exceeds a predetermined threshold (for example, can be 3nm, 5nm, 10 nm, or the like), an alert signal or the like can be issued.

The present invention also provides the use of polybenzimidazole polymer as mechanochromic material in mechanical sensing, communication, anti-counterfeiting, nondestructive inspection of material, motion detection and micro-flow monitoring. Preferably, the use comprises at least one of stress sensor, anti-counterfeiting paper, color changing textile, pressure sensor of a bandage in compression therapy, and a micro-flow monitor. In these uses, polybenzimidazoles can, for example, undergo color change in response to mechanical action, thereby giving useful information.

For example, polybenzimidazole can be coated or encapsulated on the inner surface of a microchannel; when the flow rate of fluid in the microchannel changes, the polybenzimidazole undergoes a change in stress so as to cause a change of fluorescence emission peak, and this change can be captured by a detector externally connected to the microchannel, so that the flow rate monitoring is realized. For another example, polybenzimidazole film can be disposed on the outer side of a pipe, and when the pipe undergoes a deformation, the deformation of the polybenzimidazole film is caused, so that the fluorescence emission peak is changed, and this change can be captured by a detector externally connected, thereby realizing the early warning of pipe aging.

The present invention will be described in detail below by way of Examples.

In the following Examples,

the yield of polymer is calculated by the following equation:

yield = actual amount of polymer $\div$ theoretical amount of polymer $\times$100%;

wherein the theoretical amount of polymer = the molar amount of the compound represented by formula (6) $\times$ the molar mass of the repeating unit(s) in the polymer structure.
3,3',4,4'-tetraaminobiphenyl: Sigma-Aldrich; purity 98%;
4,4'-diphenyl ether dicarboxylic acid: J&K Scientific; purity 98%;
isophthalic acid: J&K Scientific; purity 98%;
terephthalic acid: J&K Scientific; purity 98%;
4,6-pyrimidinedicarboxylic acid: J&K Scientific; purity 98%;
2,5-dihydroxyterephthalic acid: J&K Scientific; purity 98%;
3,5-pyridinedicarboxylic acid: J&K Scientific; purity 98%;
5-aminoisophthalic acid: J&K Scientific; purity 98%;

1-methylbenzimidazole: InnoChem; purity98%;
2-hydroxybenzimidazole: InnoChem; purity98%.

Example 1

A mixed monomers of 3,3',4,4'-tetraaminobiphenyl (2.14 g) and 4,4'-diphenyletherdicarboxylic acid (2.79 g) in a molar ratio of 1:1.08 was added to a methanesulfonic acid/phosphorus pentoxide solution (solvent 44 g) to form a reaction system, the reaction system was reacted at 150 °C for 3 hours to obtain a reaction solution, and the reaction solution was introduced into water to precipitate the polymer, followed by neutralization (sodium bicarbonate) and suction filtration in sequence to obtain polymer PBI-1. Wherein, the weight ratio of methanesulfonic acid to phosphorus pentoxide in the methanesulfonic acid/phosphorus pentoxide solution was 20:1, the concentration of the mixed monomers in the reaction system was 10 wt%. The yield of the polymer PBI-1 was 99%. The hydrogen nuclear magnetic resonance spectrum of PBI-1 was measured in Test Example 6 and is shown in FIG. 1. The structure was confirmed by the hydrogen nuclear magnetic resonance spectrum.

Example 2

The method of Example 1 was followed, except that 4,4'-diphenylether dicarboxylic acid was replaced by equimolar terephthalic acid, and the reaction time was 4 hours. The yield of the polymer PBI-2 was 99%. The structure was confirmed by hydrogen nuclear magnetic resonance spectrum.

Example 3

A mixed monomers of 3,3',4,4'-tetraaminobiphenyl (2.14 g), isophthalic acid (1.57 g) and 4,6-pyrimidinedicarboxylic acid (0.18 g) in a molar ratio of 1:0.945:0.105 was added to a methanesulfonic acid/phosphorus pentoxide solution (solvent 35 g) to form a reaction system, the reaction system was reacted at 150 °C for 3 hours to obtain a reaction solution, the reaction solution was introduced into water to precipitate polymer, followed by neutralization (sodium bicarbonate) and suction filtration in sequence to obtain polymer PBI-3. Wherein, the weight ratio of methanesulfonic acid to phosphorus pentoxide in the methanesulfonic acid/phosphorus pentoxide solution was 20:1, the concentration of the mixed monomers in the reaction system was 10 wt%. The yield of the polymer PBI-3 was 99%. The structure was confirmed by the hydrogen nuclear magnetic resonance spectrum.

Example 4

The method of Example 3 was followed, except that 4,6-pyrimidinedicarboxylic acid was replaced by equimolar 2,5-dihydroxyterephthalic acid and the reaction time was 4 h. The yield of the polymer PBI-4 was 99%. The structure was confirmed by hydrogen nuclear magnetic resonance spectrum.

Example 5

The method of Example 3 was followed, except that 4,6-pyrimidinedicarboxylic acid was replaced by equimolar 3,5-pyridinedicarboxylic acid and the reaction time was 5 h. The yield of the polymer PBI-5 was 99%. The structure was confirmed by hydrogen nuclear magnetic resonance spectrum.

Example 6

The method of Example 3 was followed, except that 4,6-pyrimidinedicarboxylic acid was replaced by equimolar 5-aminoisophthalic acid and the reaction time was 4 h. The yield of the polymer PBI-6 was 99%. The structure was confirmed by hydrogen nuclear magnetic resonance spectrum.

Example 7

A mixed monomers of 3,3',4,4'-tetraaminobiphenyl (2.14 g), 4,4'-diphenyletherdicarboxylic acid (2.44 g) and 5-aminoisophthalic acid (0.19 g) in a molar ratio of 1:0.945:0.105 was added to a methanesulfonic acid/phosphorus pentoxide solution (solvent 43 g) to form a reaction system, the reaction system was reacted at 150 °C for 3 hours to obtain a reaction solution, which was introduced into water to precipitate polymer, followed by neutralization (sodium bicarbonate) and suction filtration in sequence to obtain polymer PBI-7. Wherein, the weight ratio of methanesulfonic acid to phosphorus pentoxide in the methanesulfonic acid/phosphorus pentoxide solution was 20:1, the concentration of the

mixed monomers in the reaction system was 10 wt%. The yield of the polymer PBI-7 was 99%. The hydrogen nuclear magnetic resonance spectrum of PBI-7 was measured in Test Example 6 and is shown in FIG. 1. The structure was confirmed by the hydrogen nuclear magnetic resonance spectrum.

Example 8

The method of Example 7 was followed, except that the molar ratio was changed to 1:0.714:0.306, and the reaction time was 8 hours. The yield of the polymer PBI-8 was 99%. The structure was confirmed by hydrogen nuclear magnetic resonance spectrum.

Example 9

The method of Example 1 was followed, except that 4,4'-diphenylether dicarboxylic acid was replaced by equimolar isophthalic acid, and the reaction time was 5 hours. The yield of the polymer PBI-9 was 99%. The structure was confirmed by hydrogen nuclear magnetic resonance spectrum.

PBI-9 was dissolved in DMSO solvent (the concentration was 4 wt%), the solution was poured on a plate glass, and was placed in an oven at 80 °C for 10 hours, and after taken out PBI-9 film product was obtained, which was marked as PBI-9.

Example 10

A PBI film product was purchased from FuMA-Tech, Germany and was marked as PBI-10.

Example 11

A PBI film product was purchased from BASF, Germany and was marked as PBI-11.

Test Example 1

The polymers (powders) prepared in Examples 1 to 8 above were tested for mechanochromic property.

Testing method: polymer powder was placed into a solid holder (two pieces of transparent quartz plate, one of which had a recess for holding the sample) and was placed into a fluorometer (Edinburgh-steady/transient fluorescence spectrometer FLS1000) to measure and obtain the fluorescence emission spectrum. Then, the polymer powder was ground by hand in a mortar for 30s and then placed into the solid holder, and placed again into the fluorometer to measure the fluorescence emission spectrum. The shifts in strongest fluorescence emission peaks before and after grinding of polymer powders are shown in Table 1.

Test Example 2

The polymer films of Examples 9-11 were tested for mechanochromic property.

Testing method for mechanochromic property of polymer film: the film was placed into a solid holder and was placed into a fluorometer (Edinburgh-steady/transient fluorescence spectrometer FLS1000) to measure and obtain the fluorescence emission spectrum. The two ends of the film were clamped by tweezers, stretching the film for 30s under the condition of ensuring that the film was not cracked, and the film then was placed into the solid holder and placed again into the fluorometer to measure the fluorescence emission spectrum. The film thicknesses were all $40 \pm 5 \mu m$. The shifts in strongest fluorescence emission peaks before and after stretching of polymer films are shown in Table 1.

TABLE 1

| | PBI-1 | PBI-2 | PBI-3 | PBI-4 | PBI-5 | PBI-6 | PBI-7 | PBI-8 |
|---|---|---|---|---|---|---|---|---|
| shift (nm) of fluorescence emission peak of PBI after being subjected to external force | 24# | 20# | 15# | 6* | 18# | 8* | 15# | 26# |

(continued)

| | PBI-9 | PBI-10 | PBI-11 | |
|---|---|---|---|---|
| shift (nm) of fluorescence emission peak of PBI after being subjected to external force | 14* | 6# | 6# | |
| Note: "#" indicates a blue shift of the emission peak; "*" indicates a red shift of the emission peak. | | | | |

Test Example 3

The polymers prepared in the above Examples were tested for thermal stability.

Thermogravimetric analysis testing method: the test was performed in nitrogen atmosphere using a thermogravimetric analyzer, type Q500, TA, USA. The heating rate was 10 °C/min, and the temperature range was 25-700 °C. The thermal decomposition temperature $T_{d5\%}$ (temperature at which 5% by mass being lost) of the polymers are shown in Table 2.

TABLE 2

| | PBI-1 | PBI-2 | PBI-3 | PBI-4 | PBI-5 | PBI-6 | PBI-7 | PBI-8 |
|---|---|---|---|---|---|---|---|---|
| $T_{d5\%}$ (°C) | 560 | 450 | 520 | 520 | 560 | 540 | 540 | 550 |
| | PBI-9 | PBI-10 | PBI-11 | | | | | |
| $T_{d5\%}$ (°C) | 480 | 310 | 315 | | | | | |

Test Example 4

The polymers prepared in the above Examples were tested for intrinsic viscosity.

Intrinsic viscosity testing method: PBI was dissolved in concentrated sulfuric acid to prepare a sulfuric acid solution with the concentration of 0.6 g $dL^{-1}$. The sulfuric acid solution was added into a Ubbelohde viscometer, the viscometer was placed into a constant-temperature water bath at 25 °C for stabilization for 30min, and the outflow time of the solution was measured, measuring each sample for three times and the time difference being not more than 1s, wherein the average value was obtained and recorded as $t_1$, and the outflow time of concentrated sulfuric acid was recorded as $t_0$. The intrinsic viscosity [η] of PBI was calculated according to the following equations, the specific viscosity $\eta_{SP} = \frac{t_1 - t_0}{t_0}$ and the intrinsic viscosity $[\eta] = \frac{\eta_{SP} + 3\ln(1+\eta_{SP})}{4C}$, wherein C is the concentration of the PBI sulfuric acid solution. The testing results are shown in Table 3.

TABLE 3

| | PBI-1 | PBI-2 | PBI-3 | PBI-4 | PBI-5 | PBI-6 | PBI-7 |
|---|---|---|---|---|---|---|---|
| intrinsic viscosity($dL\ g^{-1}$) | 2.75 | 1.02 | 0.39 | 0.58 | 0.85 | 1.16 | 0.54 |
| | PBI-8 | PBI-9 | | | | | |
| intrinsic viscosity($dL\ g^{-1}$) | 0.30 | 1.95 | | | | | |

Test Example 5

The film prepared in Example 9 was tested for mechanical strength.

The mechanical strength testing method: 5965 universal tensile tester of Instron, USA was used, with a tensile rate of 1 mm/min and a testing temperature of 25 °C.

The modulus of the PBI-9 film is 2.5 GPa, and the nominal modulus of the purchased PBI-10 film is 4.5 GPa, and both films show excellent mechanical properties.

Test Example 6

The polymers of the Examples were tested for hydrogen nuclear magnetic resonance spectra.

The hydrogen nuclear magnetic resonance spectrum testing method: Agilent 400-MR DD2 nuclear magnetic resonance spectrometer with the frequency of 400 MHz being used, tetramethylsilane (TMS) as internal standard substance, DMSO-$d_6$ as solvent and the testing temperature being 40 °C.

The hydrogen nuclear magnetic resonance spectra of the polymers of Example 1 and Example 7 are shown in Fig. 1, wherein the proton signal at chemical shift 13.0ppm in the spectra can be assigned to the characteristic peak of active hydrogen in PBI structural unit, the proton signals at 7.30ppm and 8.28ppm can be assigned to the characteristic peaks of corresponding structural unit of 4,4'-diphenyl ether dicarboxylic acid monomer after polymerization, and the proton signals at 7.55-8.02ppm can be assigned respectively to the characteristic peaks of corresponding structural units of tetramine monomer and 5-amino isophthalic acid monomer after polymerization.

The results of hydrogen nuclear magnetic resonance spectra show that polymers PBI-1 to PBI-9 were successfully obtained.

Comparative Example

The mechanochromic properties of 1-methylbenzimidazole and 2-hydroxybenzimidazole were measured respectively according to the method of Test Example 1. The testing results showed that the strongest fluorescence emission peaks of the two had no change after grinding, namely the two did not show mechanochromic property.

The preferred embodiments of the present invention have been described above in detail, but the present invention is not limited thereto. Within the scope of the technical concept of the invention, many simple modifications can be made to the technical solutions of the invention, including combining various technical features in any other suitable ways, and those simple modifications and combinations should also be regarded as the disclosure of the invention, and all fall within the protection scope of the invention.

## Claims

1. Use of polybenzimidazole as mechanochromic material.

2. The use according to claim 1, wherein the polybenzimidazole comprises structural unit A and the structure unit A has a structure represented by formula (1):

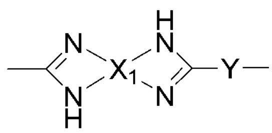

formula (1)

wherein, the $X_1$ group is a linking group provided by an aromatic hydrocarbon compound containing 1-10 benzene rings; and the Y group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$ and Y groups are each independently not substituted or substituted by at least one substituent selected from the group consisting of hydroxyl, amino, cyano, halogen, $C_1$-$C_4$ alkyl, and $C_1$-$C_4$ haloalkyl.

3. The use according to claim 1 or 2, wherein the polybenzimidazole comprises structural unit A and structural unit C, comprises structural unit A and structural unit D, or comprises structural unit A, structural unit B, structural unit C and structural unit D;
wherein the structural unit A has a structure represented by formula (1), the structural unit B has a structure represented by formula (2), the structural unit C has a structure represented by formula (1-1), and the structural unit D has a structure represented by formula (2-2):

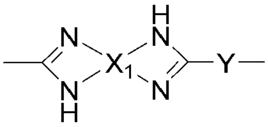 formula (1) 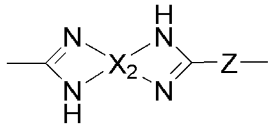 formula (2)

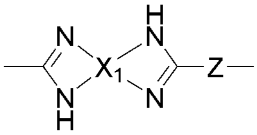 formula (1-1) 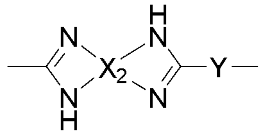 formula (2-2)

wherein, the $X_1$ and $X_2$ groups are each independently a linking group provided by an aromatic hydrocarbon compound containing 1-10 benzene rings; the Y and Z groups are each independently a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl.

**4.** The use according to claim 1 or 2, wherein the polybenzimidazole comprises structural unit A and optionally structural unit B, the structural unit A having a structure represented by formula (1) and the structural unit B having a structure represented by formula (2);

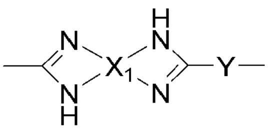 formula (1) 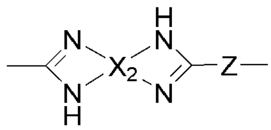 formula (2)

wherein, the $X_1$ and $X_2$ groups are each independently a linking group provided by an aromatic hydrocarbon compound containing 1-10 benzene rings; the Y and Z groups are each independently a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin, and Y is different from Z when the structural unit B exists; wherein, the $X_1$, $X_2$, Y and/or Z groups are each independently not substituted or substituted by at least one substituent selected from the group consisting of hydroxyl, amino, cyano, halogen, $C_1$-$C_4$ alkyl, and $C_1$-$C_4$ haloalkyl.

**5.** The use according to any one of claims 2 to 4, wherein the $X_1$ and $X_2$ groups are each independently a linking group provided by at least one compound selected from the group consisting of benzene, biphenyl, terphenyl, a bridged-benzenes, an iptycene, and a fused ring aromatic hydrocarbon containing 1 to 10 benzene rings; preferably, the $X_1$ and $X_2$ groups are each independently a linking group provided by at least one compound selected from the group consisting of benzene, biphenyl, a bridged-benzenes, an iptycene and naphthalene; more preferably, the bridged-benzenes has a structure represented by formula (3), formula (4) or formula (5), wherein $R_1$, $R_3$, $R_4$ are each independently selected from the group consisting of oxygen atom, sulfur atom, carbonyl, sulfonyl, methylene, and halogen-substituted methylene; and $R_2$ is selected from the group consisting of heteroatom-containing aromatic rings;

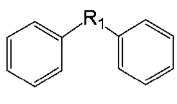 formula (3) 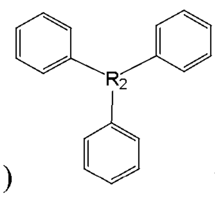 formula (4) 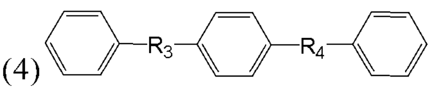 formula (5);

further preferably, the $X_1$ and $X_2$ groups are each independently selected from the following linking groups:

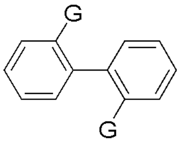 、 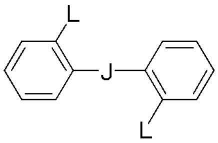 、 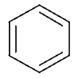 、 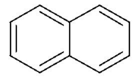 、

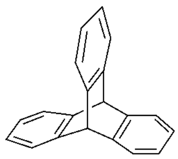 、 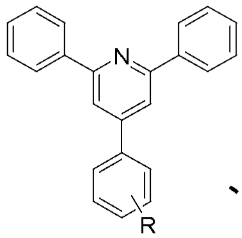 、 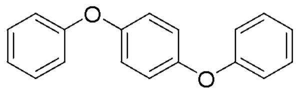 ;

wherein G is selected from hydrogen atom, methyl and trifluoromethoxy; J is selected from oxygen atom, sulfur atom, carbonyl, sulfonyl, methylene and methyl- or trifluoromethyl-substituted methylene; L is selected from bromine atom, phenyl and trifluoromethyl-substituted phenyl; and R is selected from hydrogen atom, carboxyl, hydroxyl and trifluoromethyl.

**6.** The use according to any one of claims 2-5, wherein the Y and Z groups are each independently a linking group provided by at least one compound selected from the group consisting of benzene, a 5-6 membered nitrogen-containing heterocyclic compound, an aromatic hydrocarbon compound containing 2-7 benzene rings, an aromatic hydrocarbon compound containing 1-2 nitrogen-containing heterocyclic rings and 1-5 benzene rings, a $C_4$-$C_{10}$ cycloalkane, a $C_1$-$C_8$ paraffinic hydrocarbon and a $C_2$-$C_8$ olefin;
preferably, the Y and Z groups are each independently a linking group selected from:

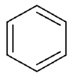 、 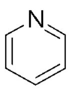 、 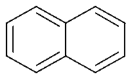 、 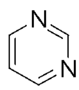 、 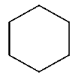 、 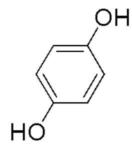 、 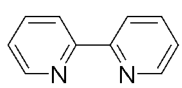 、 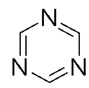 、

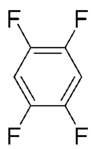 、 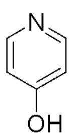 、 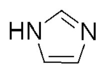 、 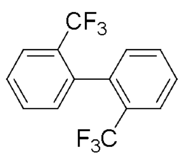 、 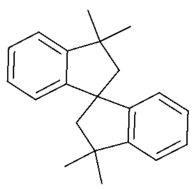 、 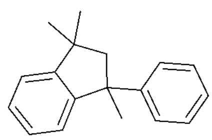 、

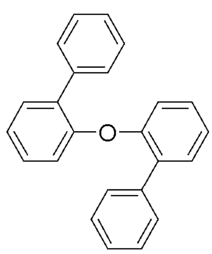 、 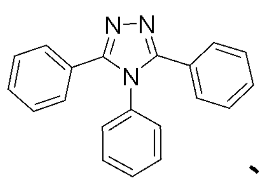 、 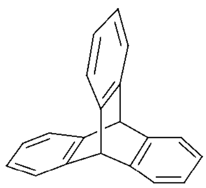 、 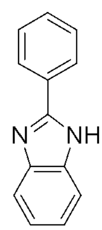 、 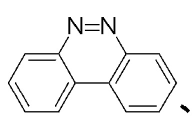 、

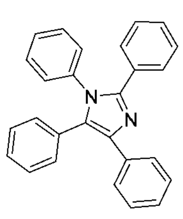 、 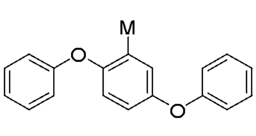 、 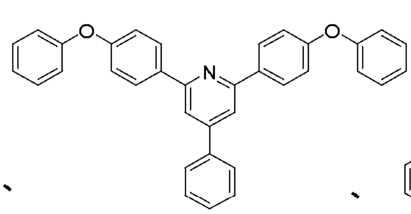 、 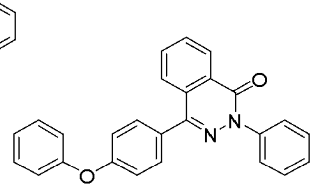 、

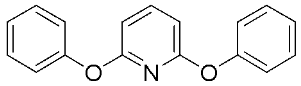 、 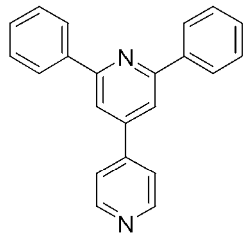 、 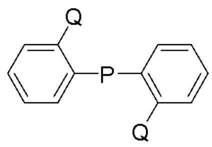 、 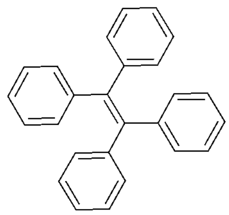 、

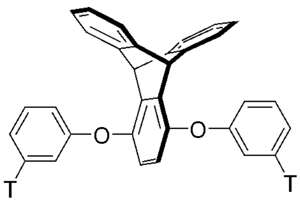 、 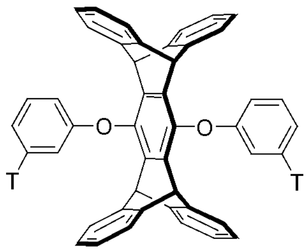 、 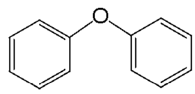 、

$C_1$-$C_8$ paraffinic hydrocarbon group;
wherein M is selected from the group consisting of hydrogen atom, cyano, methyl, phenyl, and methyl- or trifluoromethyl-substituted phenyl; P is selected from the group consisting of oxygen atom, sulfur atom, carbonyl, sulfonyl, cyclobutyl, halogen-substituted cyclobutyl, methylene and halogen-substituted methylene; Q is selected from the group consisting of bromine atom, phenyl and trifluoromethyl-substituted phenyl; and T is selected from the group consisting of hydrogen atom and methyl.

**7.** The use according to claim 1, wherein the polybenzimidazole is poly(2,5-benzimidazole).

**8.** The use according to any one of claims 1 to 7, wherein,

the polybenzimidazole has an intrinsic viscosity of 0.15-3.0 dL $g^{-1}$; and/or
the molar content ratio of $X_2$ group to $X_1$ group in the polybenzimidazole is 0-9999, preferably 0-99; and/or
the molar content ratio of the Z group to the Y group in the polybenzimidazole is 0-9999, preferably 0-99.

**9.** The use according to any one of claims 1 to 8, wherein the polybenzimidazole is used as a mechanochromic material in mechanical sensing, communication, anti-counterfeiting, nondestructive inspection of material, motion detection, and micro-flow monitoring; preferably, polybenzimidazole is used as a mechanochromic material in stress sensor, anti-counterfeiting paper, color-changing textile, pressure sensor of a bandage in compression therapy and micro-flow monitor.

**10.** The use according to any one of claims 1 to 9, wherein the polybenzimidazole undergoes a blue shift in fluorescence emission peak after stimulated by an external force.

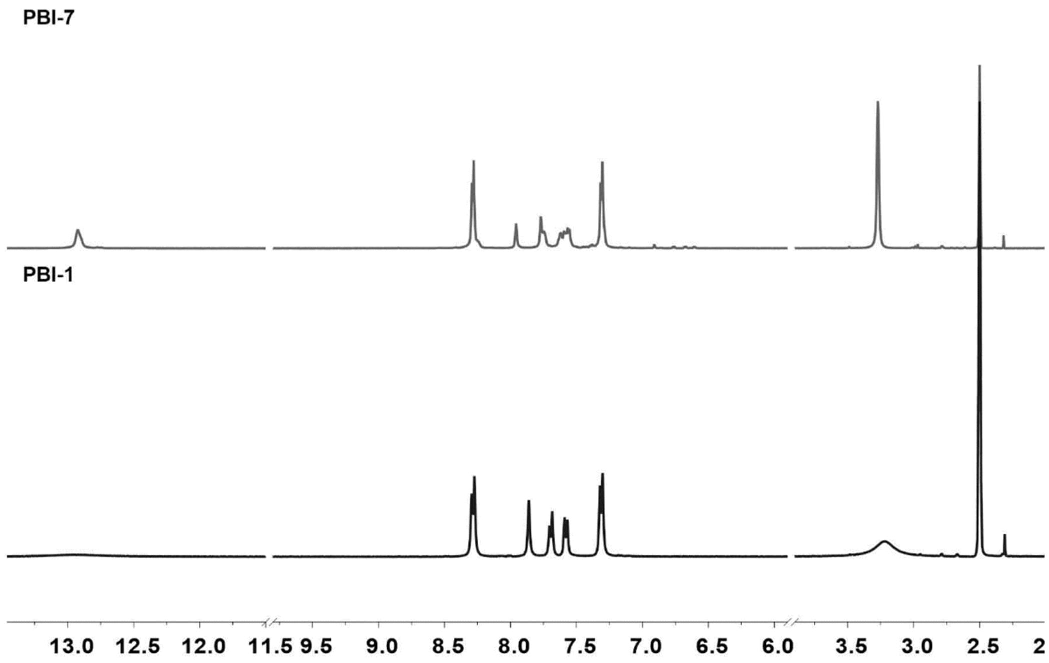
PBI-7
PBI-1
13.0 12.5 12.0 11.5 9.5 9.0 8.5 8.0 7.5 7.0 6.5 6.0 3.5 3.0 2.5 2

FIG.1

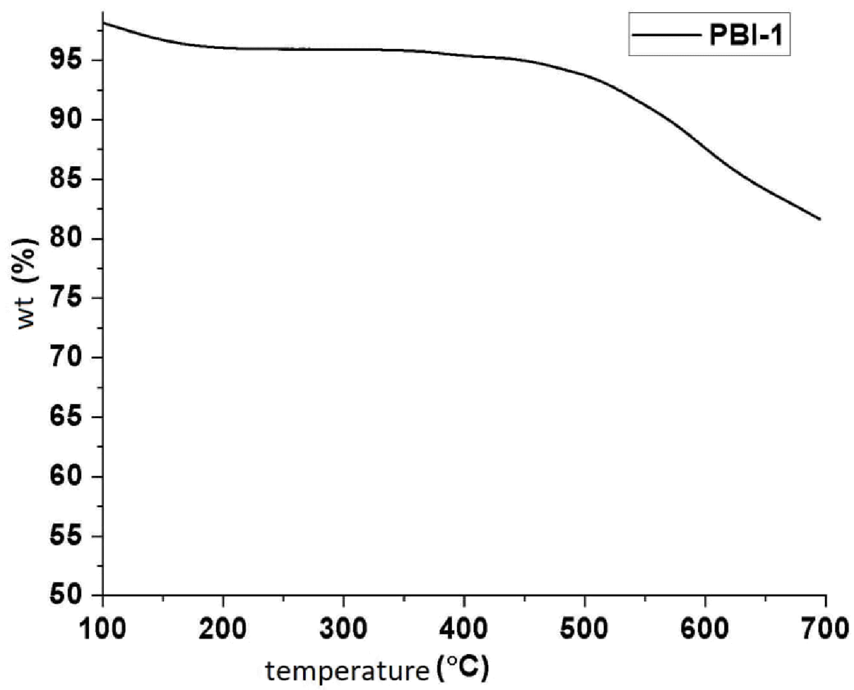
PBI-1
wt (%)
95
90
85
80
75
70
65
60
55
50
100 200 300 400 500 600 700
temperature (°C)

FIG.2

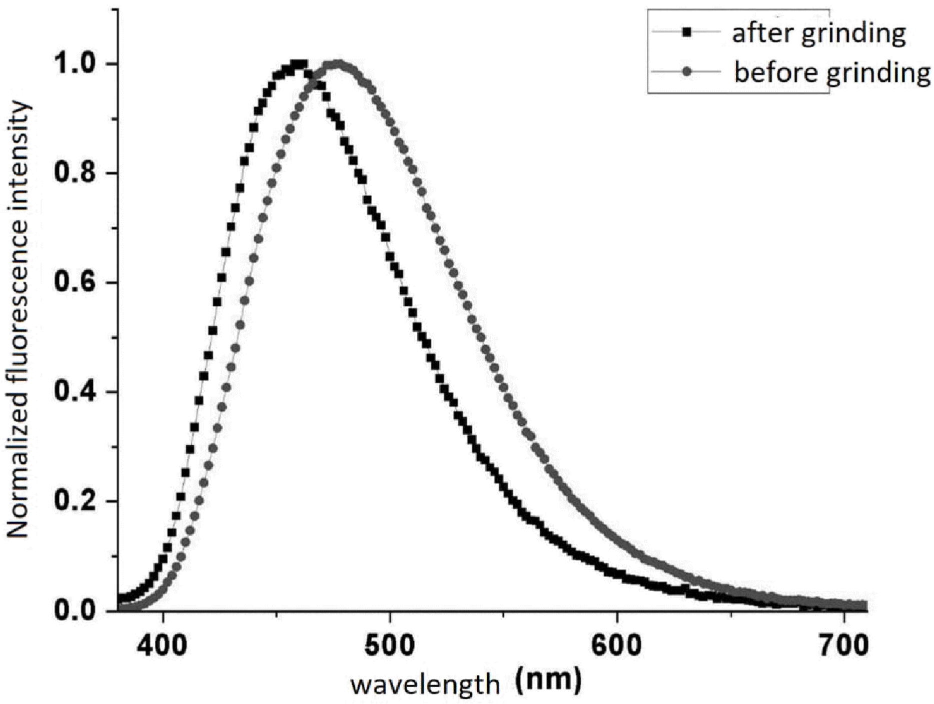
after grinding
before grinding
1.0
0.8
0.6
0.4
0.2
0.0
Normalized fluorescence intensity
400
500
600
700
wavelength (nm)

FIG.3

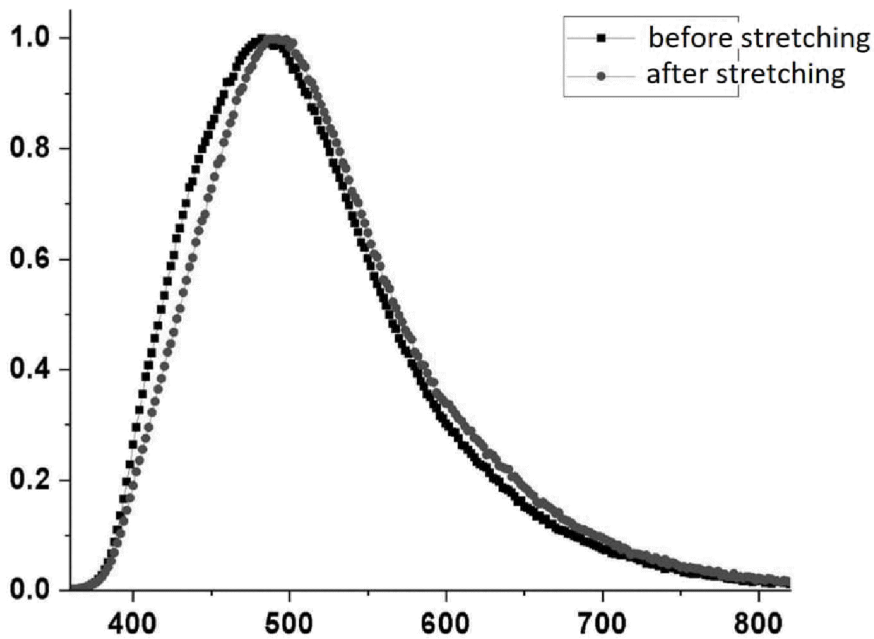
before stretching
after stretching
1.0
0.8
0.6
0.4
0.2
0.0
400
500
600
700
800

FIG.4

## INTERNATIONAL SEARCH REPORT

International application No.
**PCT/CN2024/109374**

**A. CLASSIFICATION OF SUBJECT MATTER**

C08G73/18(2006.01)i; G01N21/64(2006.01)i; C09K9/02(2006.01)i

According to International Patent Classification (IPC) or to both national classification and IPC

**B. FIELDS SEARCHED**

Minimum documentation searched (classification system followed by classification symbols)

IPC: C08G73/-; G01N21/-; C09K9/-

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, CNKI, ISI_Web of Science: +苯并咪唑+, 聚苯并咪唑, PBI, ABPBI, 聚合物, 共聚物, 均聚物, 高分子, 变色, 蓝移, 红移, 发光, 机械, 机械+, 挤压, 拉伸, 力致, 压致, 研磨, 按压, 冲击, +benzimidazole, polymer, copolymer, homopolymer, macromolecule, discoloration, blue 2d shifted, red 2d shifted, mechanical, extrusion, stretch +, force induced, pressure induced, grind+, press+, impact, mechanochromism, mechanofluorochromism

**C. DOCUMENTS CONSIDERED TO BE RELEVANT**

| Category* | Citation of document, with indication, where appropriate, of the relevant passages | Relevant to claim No. |
|---|---|---|
| A | CN 114315732 A (WUYI UNIVERSITY) 12 April 2022 (2022-04-12)<br>description, paragraph [0028] | 1-10 |
| A | CN 112430306 A (NANJING INSTITUTE OF TECHNOLOGY) 02 March 2021 (2021-03-02)<br>claim 1 | 1-10 |
| A | CN 115490859 A (CHINA PETROCHEMICAL CORP. et al.) 20 December 2022 (2022-12-20)<br>entire document | 1-10 |
| A | JP 2014058606 A (KYUSHU UNIVERSITY) 03 April 2014 (2014-04-03)<br>entire document | 1-10 |
| A | JP 2016210991 A (TOKYO INSTITUTE OF TECHNOLOGY) 15 December 2016 (2016-12-15)<br>entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2024** | **12 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/109374**

**C. DOCUMENTS CONSIDERED TO BE RELEVANT**

| Category* | Citation of document, with indication, where appropriate, of the relevant passages | Relevant to claim No. |
|---|---|---|
| A | KR 102491823 B1 (NBST CO., LTD.) 26 January 2023 (2023-01-26)<br>entire document | 1-10 |

Form PCT/ISA/210 (second sheet) (July 2022)

**INTERNATIONAL SEARCH REPORT**
**Information on patent family members**

International application No.
**PCT/CN2024/109374**

| Patent document cited in search report | Publication date (day/month/year) | Patent family member(s) | Publication date (day/month/year) |
|---|---|---|---|
| CN 114315732 A | 12 April 2022 | None | |
| CN 112430306 A | 02 March 2021 | None | |
| CN 115490859 A | 20 December 2022 | None | |
| JP 2014058606 A | 03 April 2014 | JP 6241702 B2 | 06 December 2017 |
| JP 2016210991 A | 15 December 2016 | JP 6758702 B2 | 23 September 2020 |
| KR 102491823 B1 | 26 January 2023 | None | |

Form PCT/ISA/210 (patent family annex) (July 2022)